# EUROPÄISCHE PATENTSCHRIFT

(11) **EP 4 049 606 B1**
(45) Veröffentlichungstag und Bekanntmachung des Hinweises auf die Patenterteilung: **23.09.2026**
(21) Anmeldenummer: 22156696.1
(22) Anmeldetag: 15.02.2022
(51) Int. Cl.: A61B 18/14

(54) **ELEKTROCHIRURGISCHES HANDGERÄT UND KONTAKTKÖRPER FÜR ELEKTROCHIRURGISCHES HANDGERÄT**
ELECTROSURGICAL INSTRUMENT AND CONTACT BODY FOR ELECTROSURGICAL INSTRUMENT
APPAREIL ÉLECTROCHIRURGICAL PORTATIF ET CORPS DE CONTACT POUR APPAREILS ELECTROCHIRURGICAUX PORTATIFS

(30) Priorität: 26.02.2021 DE 102021104612
(43) Veröffentlichungstag der Anmeldung: 31.08.2022
(73) Patentinhaber: Olympus Winter & Ibe GmbH, 22045 Hamburg (DE)
(72) Erfinder: BROCKMANN, Christian, 21279 Hollenstedt (DE); KNOPF, Christoph, 23617 Stockelsdorf (DE)
(74) Vertreter: Hoener, Matthias

(56) Entgegenhaltungen:
- WO-A1-95/19142
- US-A- 6 105 581
- US-A1- 2001 011 161
- US-B2- 6 974 458

## Beschreibung

Die Erfindung betrifft einen Kontaktkörper für elektrochirurgische Handgeräte gemäß dem Oberbegriff des Anspruchs 1. Außerdem betrifft die Erfindung ein elektrochirurgisches Handgerät gemäß dem Anspruch 8.

Elektrochirurgische Handgeräte, wie beispielsweise Resektoskope, werden hauptsächlich für endoskopische Anwendungen in der Urologie oder in der Gynäkologie eingesetzt und dort vorzugsweise für die Behandlung im Bereich der Blase, Gebärmutter oder der Prostata. Das Einsatzgebiet dieser Instrumente ist jedoch nicht auf diese Bereiche des menschlichen Körpers beschränkt, sondern umfasst vielmehr auch die Behandlung weiterer Organe im menschlichen Unterleib.

Aus dem Stand der Technik sind die Dokumente US2001/011161A1, WO95/19142A1, US6974458B2 und US6105581A bekannt.

Die hier beschriebenen gattungsgemäßen Instrumente, wie beispielsweise Resektoskope, weisen standardmäßig einen Transporteur auf. Zur Behandlung des erkrankten Gewebes wird das Resektoskop mit einem langgestreckten Schaft durch eine Öffnung in den Körper des Patienten eingeführt. In diesem Schaftrohr können verschiedene medizinische Instrumente zur Behandlung und/oder zur Untersuchung des Patienten angeordnet sein. Zum Beispiel kann etwa für die Hochfrequenzchirurgie eine mit hochfrequentem Wechselstrom beaufschlagbare Elektrode, die an einem distalen Ende eines Elektrodenträgers positioniert ist, durch den Schaft geführt werden. Für die Manipulation bzw. das Schneiden des Gewebes ist der Elektrodenträger mit der Elektrode relativ zum Schaftrohr sowie entlang einer Schaftachse verschiebbar, so dass durch Entlangbewegen der Elektrode an oder durch das Gewebe dieses manipuliert wird.

Der Elektrodenträger ist des Weiteren mit seinem proximalen Ende an den Transporteur gekoppelt. Durch diese Handhabung des Transporteurs wird die Schneidbewegung der Elektrode ermöglicht. Der Transporteur weist einen bewegbar gelagerten Kontaktkörper auf, der auch als Schlitten bezeichnet wird. An diesem Kontaktkörper kann der Elektrodenträger mit einem elektrischen Kontakt mechanisch lösbar gekoppelt sein. Über diese mechanische Verbindung ist der Elektrodenträger bzw. die Elektrode auch mit elektrischer Energie versorgbar. Dazu weist der Kontaktkörper eine sacklochartige Bohrung bzw. Öffnung auf, in die ein elektrischer Kontakt des Elektrodenträgers für die lösbare Verbindung führbar ist. Diese Öffnung bzw. dieses Sackloch ist derart ausgebildet, dass über einen angrenzenden Steckverbinder die HF-Spannung beaufschlagbar ist. Dazu wird üblicherweise ein Stecker in die Buchse gesteckt, der wiederum über eine Leitung bzw. ein Kabel mit einem HF-Generator verbindbar ist.

Die Betätigung bzw. der Längsverschub des Transporteurs, also auch der Elektrode, wird durch einen Operateur ausgeführt. Dazu ist dem Transporteur eine Griffeinheit mit einem ersten Griffmittel und einem zweiten Griffmittel zugeordnet. Der Operateur erfasst zum Betätigen des Transporteurs das erste Griffmittel, als auch das zweite Griffmittel, das eine Fingereinheit bzw. einen Daumenring aufweisen kann. Das erste Griffmittel kann an einem feststehenden Hauptkörper des Transporteurs befestigt sein. Das zweite Griffmittel kann an dem Kontaktkörper befestigt sein.

Die Bewegung des Transporteurs erfolgt gegen eine Federspannkraft einer Feder, die beim gattungsgemäßen Transporteur üblicherweise als Blattfeder oder als Schenkelfeder ausgebildet ist. Diese Feder ist mit einem Ende an dem Kontaktkörper bzw. dem Schlitten und mit dem anderen Ende an einem Endkörper bzw. einer Optikführungsplatte eines Verstärkungsrohres befestigt. Die Art der Federn bzw. die Art der Betätigung dieses Federmechanismus richtet sich danach, ob es sich bei dem Transporteur um einen aktiven oder einen passiven Transporteur handelt. Während die Feder bei einem aktiven Transporteur als Druckfeder ausgebildet ist, ist sie bei einem passiven Transporteur als Zugfeder ausgebildet.

Üblicherweise erfolgt das Schneiden der Elektrode durch eine rückziehende Bewegung des Transporteurs. Beim aktiven Transporteur wird dazu die Elektrode gegen die Federkraft der Feder zurückgezogen (in proximale Richtung). Beim passiven Transporteur hingegen wird die Elektrode zunächst entgegen der Federkraft vorgeschoben (in distale Richtung), um dann bei der durch die Entlastung der Feder hervorgerufenen Rückbewegung (in proximale Richtung) durch das Gewebe zu schneiden.

Durch den Schaft der hier beschriebenen Instrumente ist außerdem eine Optik führbar. Die stabartige bzw. schaftartige Optik wird von dem proximalen Ende her durch ein Verstärkungsrohr des Transporteurs in den Schaft geführt. Es sind Ausführungsbeispiele bekannt, bei denen die Optik als Stablinsensystem oder als Glasfaser von einem proximalen Ende durch den Schaft zum distalen Ende geführt wird. Das distale Ende der Optik ist direkt auf den zu operierenden Bereich bzw. den Wirkort der Elektrode gerichtet. Am proximalen Ende der Optik kann der Operateur durch ein Okular oder eine Kamera die Behandlung beobachten.

Der mindestens eine elektrische Kontakt des Elektrodensystems wird dann mit dem Kontaktkörper verbunden und sodann durch bzw. in den Schafft geschoben. Durch Betätigung des zweiten Griffmittels relativ zu dem ersten Griffmittel lässt sich so der Kontaktkörper zusammen mit dem Elektrodenträger über ein Verstärkungsrohr entlang der Schaftachse vor und zurück bewegen.

Am proximalen Ende des Verstärkungsrohres ist dieses mit einer Optikführungsplatte verschweißt. Der Kontaktkörper ist beweglich auf dem Verstärkungsrohr angeordnet. Dazu weist der Kontaktkörper eine entsprechende Bohrung für das Verstärkungsrohr parallel zu der Schaftachse auf. Diese Bohrung ist derart bemessen, dass sich der Kontaktkörper bzw. der Schlitten leicht über das Verstärkungsrohr schieben lässt.

Als besonders nachteilig hat sich die Reinigbarkeit der Kontaktaufnahme bzw. des Sackloches für den Kontakt des Elektrodenträgers erwiesen. Insbesondere durch den geringen Lochdurchmesser als auch Verwendung von elektrischen Tulpenkontakten innerhalb des Sacklochs gestaltet sich die Reinigung als sehr schwer. Selbst durch eine maschinelle Reinigung kann nicht garantiert werden, dass der notwendige Reinigungsgrad erreicht wird. Insbesondere Rückstände von Flüssigkeiten oder Partikel, die während der Behandlung oder auch während des Reinigungsprozesses in die Aufnahme gelangen, lassen sich nur schwer oder gar nicht entfernen. Neben dem Problem der ungenügenden Aufbereitung bzw. Reinigung können sowohl in der Aufnahme verbleibende Partikel als auch Flüssigkeit zu Kontaktproblemen zwischen dem Elektrodenträger und dem Kontakt in der Aufnahme führen.

Davon ausgehend liegt der Erfindung die Aufgabe zu Grunde, ein elektrochirurgisches Handgerät sowie einen Kontaktkörper zu schaffen, der besonders einfach, gründlich sowie zeiteffizient reinigbar ist.

Ein Kontaktkörper zur Lösung dieser Aufgabe weist die Merkmale des Anspruchs 1 auf. Demnach ist es vorgesehen, dass der Kontaktkörper für ein elektrochirurgisches Handgerät mindestens eine Aufnahme aufweist, die als durchgehende Bohrung durch den Kontaktkörper ausgebildet ist. Diese durchgehende, sich von einer Stirnseite des Kontaktkörpers zu einer gegenüberliegenden Stirnseite des Kontaktkörpers erschreckende, Aufnahme lässt sich besonders gründlich sowie einfach reinigen. Diese kanalartige Bohrung durch den gesamten Kontaktkörper kann besonders gründlich mit einem Reinigungsmedium durchgespült werden und zwar derart, dass weder Partikel noch eine Restflüssigkeit in der Aufnahme verbleibt. Eine derart ausgewählte Aufnahme für den elektrischen Kontakt lässt sich auch besonders gut trocknen, sodass nach dem Reinigungsprozess der Innenraum der Aufnahme rückstandslos aufbereitet ist.

Vorzugsweise ist es weiter vorgesehen, dass der Kontaktkörper zwei Aufnahmen aufweist, die jeweils als durchgehende Bohrungen durch den gesamten Kontaktkörper ausgebildet sind. Ein derart ausgebildeter Kontaktkörper ist ausgelegt für die Aufnahme bzw. elektrische Kontaktierung eines Elektrodenträgers mit zwei elektrischen Kontakten. Bei diesen elektrischen Kontakten, die jeweils, insbesondere durch Elektrodenhüllrohre, zu der distal angeordneten Elektrode führen, kann es sich um einen Aktiv-Kontakt und einen Return-Kontakt bzw. um die Aktiv-Elektrode und die Return-Elektrode handeln.

Bevorzugterweise ist es vorgesehen, dass die beiden Aufnahmen für die beiden vorgenannten elektrischen Kontakte parallel zueinander sowie parallel zu einer Längsachse des Handgerätes bzw. des Elektrodenträgers ausgerichtet sind und in einer gemeinsamen Ebene liegen. Durch diese Symmetrie gestaltet sich nicht nur die Bauform des Handgerätes und des Elektronenträgers als besonders kompakt und raumsparend sondern auch die Form des Kontaktkörpers. Insbesondere die parallele Anordnung der Aufnahmen zu einer Schaftachse und somit gegebenenfalls auch zu einer Bohrung zur Aufnahme eines Verstärkungsrohres für die Optik, gestaltet sich das Koppeln bzw. das Entkoppeln des Elektrodenträgers mit dem Kontaktkörper als besonders vorteilhaft. So kann durch diese relative Anordnung nicht nur eine verlässliche Kontaktierung sondern auch eine einfach zu bedienende Kontaktierung realisiert werden. Bereits durch geringe Kraftausübung lassen sich die beiden Kontakte in die Aufnahmen einführen als auch wieder entnehmen. Gleichermaßen ist die mechanische Kopplung der Kontakte in dem Kontaktkörper ausreichend stark, um auch Kräften, die während der Behandlung parallel oder quer zur Längsachse auftreten, standzuhalten.

Insbesondere sieht es die Erfindung vor, dass mindestens eine Aufnahme einer Innenwandung einen elektrischen Kontakt aufweist, der über einen Steckverbinder, insbesondere über eine Steckerbuchse, und eine elektrische Verbindung mit einem Generator verbindbar ist, wobei der Steckverbinder vorzugsweise in den Kontaktkörper integriert ist. Dabei kann der Steckverbinder senkrecht oder parallel zu der Aufnahme in dem Kontaktkörper angeordnet sein. Vorzugsweise wird der Steckverbinder von unten durch den Kontaktkörper hindurch an die kanalartige Aufnahme herangeführt. Zusätzlich kann es besonders vorteilhaft sein, gegebenenfalls beide Aufnahmen durch entsprechende externe Stecker zu kontaktieren. Durch diese Kontaktierung der beiden Aufnahmen lässt sich ein besonders definiertes sowie stabiles elektrisches Potenzial an den Elektrodenträgerenden erzeugen. Gleichermaßen ist denkbar, dass die beiden Stecker bzw. Steckverbindungen von der proximalen Stirnseite aus in die mindestens eine Aufnahme des Kontaktkörpers geführt werden. Alternativ ist es außerdem denkbar, dass ein Kabel aus dem Kontaktkörper herausgeführt wird, an dem ein Steckverbinder angeordnet ist.

Weiter kann es erfindungsgemäß vorgesehen sein, dass der elektrische Kontakt innerhalb der mindestens einen Aufnahme als zylinderförmiger, hohlzylinder- oder ringförmiger elektrischer Kontakt, vorzugsweise als Lamellenkontakt oder Spiralfederkontakt, ausgebildet ist. Durch diese Kontaktarten lässt sich die elektrische Kontaktierung der Kontakte des Elektrodenträgers besonders zuverlässig herstellen. Darüber hinaus ist es auch denkbar, dass den Aufnahmen eine andere Kontaktform zur elektrischen Kontaktierung zugeordnet ist. Ein besonders vorteilhaftes Ausführungsbeispiel der Erfindung kann es weiter vorsehen, dass ein Abstand des mindestens einen Kontaktes zu einer Stirnseite des Kontaktkörpers, insbesondere zu einem Austritt der mindestens einen Aufnahme, mindestens 2 mm, bevorzugst mindestens 4 mm, insbesondere mindestens 6 mm, beträgt.

Ein weiteres bevorzugtes Ausführungsbeispiel der Erfindung kann es vorgesehen, dass sich in mindestens eine Aufnahme ein mechanisches Rastmittel erstreckt zur lösbaren Kopplung mindestens eines proximalen Endes des Elektrodenträgers, wobei das Rastmittel über ein Handhabungsmittel, insbesondere einen federvorgespannten Knopf, betätigbar, vorzugsweise arretierbar und/oder lösbar, ist. Vorzugsweise wird nur einer der beiden Kontakte des Elektrodenträgers mit dem Rastmittel arretiert, um einen elektrischen Kurzschluss zwischen den beiden Kontakten zu vermeiden. Es ist denkbar, dass der entsprechende proximale Bereich an dem Elektrodenträger, an dem die Rastung vorgenommen wird, eine Verformung im Querschnitt, wie beispielsweise eine Kerbe oder eine Ausnehmung aufweist, in die das Rastmittel rastend eingreifen kann. Diese Rastverbindung ist derart ausgebildet, dass sie einhändig von dem Operateur durchführbar.

Darüber hinaus kann eine vorteilhafte Weiterbildung darin gesehen werden, dass die Bohrung der mindestens einen Aufnahme an einem distalen Ausgang trichterartig ausgeführt ist, insbesondere dass eine Stirnseite des Kontaktkörpers um die Bohrung der mindestens einen Aufnahme eine trichterartige Senkung aufweist. Durch diese trichterartige Form des Eingangsbereichs der Aufnahme wird das Einführen der Kontakte des Elektrodenträgers in die Aufnahmen erleichtert. Dabei ist es ausreichend, dass beim Zusammenführen des Elektrodenträgers mit dem Kontaktkörper die beiden proximalen Enden des Elektrodenträgers in die Trichter geführt werden. Die schräge Form der Trichterinnenwände führen die Kontakte dann direkt in die Aufnahmen. Dies erleichtert die Kontaktierung des Elektrodenträgers insbesondere während der Operation.

Weiter kann es erfindungsgemäß vorgesehen sein, dass in den Aufnahmen bzw. in den Bohrungen vor oder hinter den elektrischen Kontakten eine Dichtung, insbesondere eine Fluiddichtung, angeordnet ist. Durch diese Dichtung kann vermieden werden, dass während der Behandlung Spülflüssigkeit aus dem Schaft durch die Aufnahme des Kontaktkörpers fließt und gegebenenfalls zu einem elektrischen Kurzschluss führt. Durch diese Dichtung wird die Flüssigkeit wenigstens größtenteils aus der Aufnahme herausgehalten. Besonders bevorzugt ist diese Dichtung direkt am distalen Ende der Aufnahmen angeordnet.

Es hat sich herausgestellt, dass Kunststoff, insbesondere PTFE, für den Kontaktkörper aufgrund der Materialeigenschaften wie beispielsweise geringer Geleitswiderstand, hoher elektrischer Widerstand, glatte Oberfläche sowie gute Bearbeitbarkeit besonders vorteilhaft ist. Gleichermaßen ist es jedoch auch denkbar, dass der Kontaktkörper aus einem anderen Fluorpolymer wie beispielsweise PFA herstellbar ist. Auch PEEK hat sich als vorteilhaft erwiesen. Diese Materialien eignen sich auch deshalb besonders gut, da sie besonders gründlich reinigbar sind.

Eine weitere Lösung der eingangs genannten Aufgabe wird durch die Merkmale des Anspruchs 8 beschrieben. Demnach ist es vorgesehen, dass ein elektrochirurgisches Handgerät, wie beispielsweise ein Resektoskop, mit einem Elektrodenträger, der an einem distalen Ende eine Elektrode und an einem proximalen Ende mindestens einen elektrischen Kontakt aufweist, einen Kontaktkörper nach mindestens einem der Ansprüche 1 - 7 aufweist, in dem das mindestens eine proximale Ende des Elektrodenträgers kontaktierbar ist.

Bevorzugterweise ist es vorgesehen, dass wenigstens eine Komponente, insbesondere ein Anschlag, des Handgerätes, der in proximale oder distale Richtung an den Kontaktkörper angeordnet ist oder an den Kontaktkörper anschlägt, aus einem elektrisch nicht leitenden Material besteht oder eine elektrische nicht leitende Schicht, vorzugsweise eine Beschichtung, aufweist. Dadurch kann verhindert werden, dass Spülflüssigkeit, während der Behandlung des Patienten durch die kanalartige Aufnahme tritt und eine elektrische Verbindung zwischen dem Kontakt des Elektrodenträgers und beispielsweise dem rückleitenden, elektrischen Potential erzeugt. Als Alternative ist eine Verlängerung der Aufnahme möglich, indem z.B. das proximale Ende der Aufnahme nicht an der proximalen Stirnseite sondern z.B. an der Unterseite austritt.

Weiter ist es denkbar, dass eine elektrisch leitfähige Komponente, insbesondere ein Anschlag, des Handgerätes durch einen Luftspalt von dem Kontaktkörper, vorzugsweise einem Austritt mindestens einer kanalartigen Aufnahme des Kontaktkörpers, getrennt ist, wobei der Luftspalt eine Breite von mindestens 1 mm, insbesondere mindestens 2 mm, aufweist. Beispielsweise ist es denkbar, dass eine Anschlagsfläche radial von dem Austritt der mindestens einen kanalartigen Aufnahme des Kontaktkörpers beabstandet ist.

Darüber hinaus ist es vorzuweise vorgesehen, dass ein Abstand zwischen der Elektrode und einem distalen Ende eines Schaftes, insbesondere der Optik oder einem Elektrodenhüllrohr, geringer ist als ein Abstand zwischen einem proximalen Ende des Elektrodenträgers und einer proximalen Stirnseite des Kontaktkörpers und/oder geringer ist als ein Abstand zwischen einem elektrischen Kontakt und wenigstens einer Stirnseite des Kontaktkörpers. Dadurch soll sichergestellt werden, dass während der Behandlung ein Plasma zwischen der Elektrode und der Neutralelektrode zündet und nicht im Kontaktkörper zwischen dem proximalen Ende des Elektrodenträgers und der proximalen Stirnseite des Kontaktkörpers.

Ein bevorzugtes Ausführungsbeispiel der Erfindung wird im Folgenden anhand der Zeichnung näher erläutert. In dieser zeigen:
- Fig. 1: eine schematische Darstellung eines Resektoskopes,
- Fig. 2: eine Seitenansicht eines Kontaktkörpers,
- Fig. 3: eine Sicht auf eine Stirnseite des Kontaktkörpers gemäß Fig. 2, und
- Fig. 4: ein Schnitt durch den Kontaktkörper gemäß Fig. 2.

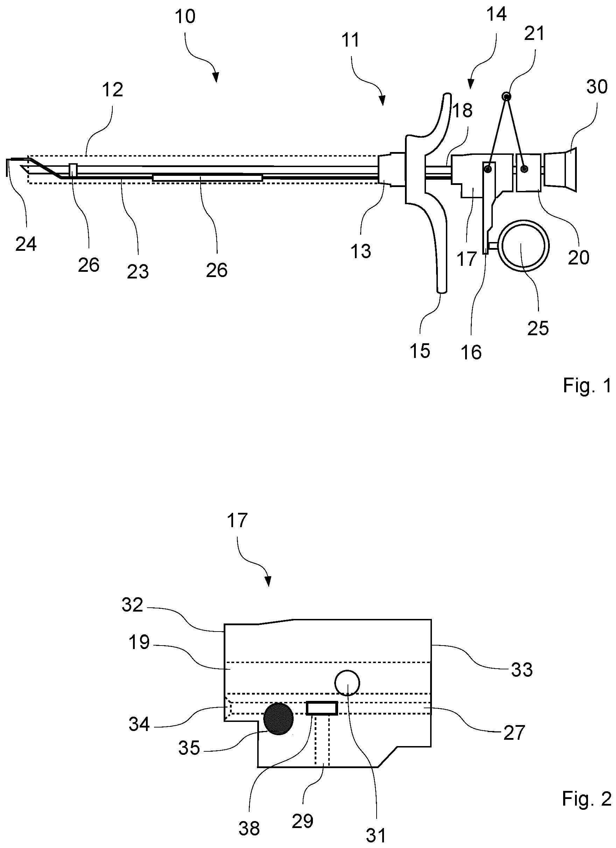

Ein mögliches Ausführungsbeispiel eines elektrochirurgischen Handgerätes, nämlich eines Resektoskopes 10, ist stark schematisiert in der Fig. 1 dargestellt. Das Resektoskop 10 weist einen Transporteur 11 auf, an dem ein langgestreckter, rohrartiger Schaft 12 befestigbar ist. Dieser Schaft 12 ist in der Fig. 1 schraffiert dargestellt und ist mit einem proximalen Ende an einem Hauptkörper 13 des Transporteurs 11 befestigt.

Der Transporteur 11 weist neben dem Hauptkörper 13 eine Griffeinheit 14 auf. Diese Griffeinheit 14 verfügt über ein erstes Griffmittel 15 und ein zweites Griffmittel 16. Während das erste Griffmittel 15 fest an dem Hauptkörper 13 angeordnet ist, ist bei dem hier dargestellten Ausführungsbeispiel des Transporteurs 11 das zweite Griffmittel 16 einem Kontaktkörper 17 zugeordnet. Dabei ist es denkbar, dass das zweite Griffmittel 16 an dem Kontaktkörper 17 festgeschraubt ist. Dazu weist der Kontaktkörper 17 in einer Wandung eine entsprechende Bohrung 31 auf.

Der Kontaktkörper 17 wird gleitend auf einer rohrartigen Optikführung 18 geführt. Dazu weist der Kontaktkörper 17 eine Bohrung 19 auf, deren Durchmesser geringfügig größer ist als ein Durchmesser der Optikführung 18. Da sich der Kontaktkörper 17 auf der Optikführung 18 entlang einer Längsrichtung des Resektoskops 10 bzw. einer Längsachse des Schaftes 12 hin und her bewegen lässt, wird der Kontaktkörper 17 auch als Schlitten bezeichnet.

Während die Optikführung 18 mit einem distalen Ende mit dem Hauptkörper 13 oder einem Innenrohr 22 verbunden ist, ist an einem proximalen Ende der Optikführung 18 eine Optikführungsplatte 20 befestigt. Die rohrartige Optikführung 18 erstreckt sich durch die Optikführungsplatte 20, sodass die Optikführung 18 von proximal aus zugänglich ist.

Das zweite Griffmittel 16 bzw. der Kontaktkörper 17 sind über ein Federelement 21 mit der Optikführungsplatte 20 verbunden. Bei diesem Federelement 21 kann es sich je nach Bauart des Transporteurs 11 um eine Zugfeder oder eine Druckfeder handeln.

Ausgehend von dem Hauptkörper 13 erstreckt sich ein rohrartiges Innenrohr 22 in distale Richtung. Dieses Innenrohr 22 kann sich auch in proximale Richtung durch den Hauptkörper 13 erstrecken und mit der Optikführung 18 verbunden werden.

Parallel zu dem Innenrohr 22 erstreckt sich ein Elektrodenträger 23. Dieser Elektrodenträger 23 wird durch den Hauptkörper 13 geführt und ist mit wenigstens einem proximalen Kontakt mechanisch mit dem Kontaktkörper 17 lösbar gekoppelt. An einem distalen Ende weist der Elektrodenträger 23 eine Elektrode 24 auf. Diese Elektrode 24 ist mit einer elektrischen HF-Spannung beaufschlagbar. Mittels sich an der Elektrode 24 bildende thermische Energie lässt sich das erkrankte Gewebe manipulieren bzw. schneiden. Dazu bewegt der Operateur das einen Daumenring 25 aufweisende zweite Griffmittel 16 relativ zu dem ersten Griffmittel 15. Zur Stabilisierung des Elektrodenträgers 23 kann dieses an dem Innenrohr 22 durch Führungen 26 geführt werden.

Für die Beaufschlagung der Elektrode 24 mit der HF-Spannung lässt sich eine Aufnahme 27 des proximalen Kontaktes des Elektrodenträgers 23 elektrisch kontaktieren. Dazu weist der Kontaktkörper 17 mindestens einen Steckverbinder 29, insbesondere eine Steckerbuchse, auf. Dieser der Steckverbinder 29 befindet sich im elektrischen Kontakt mit wenigstens einem Teil einer Innenwandung der Aufnahme 27. Über einen hier nicht dargestellten Stecker lässt sich so der Kontaktkörper 17 über ein Kabel mit einem HF-Generator verbinden.

Für die Durchführung des Eingriffs wird durch das Innenrohr 22 bzw. Optikführung 18 eine stabartige Optik geführt. Ein hier nicht sichtbares distales Ende dieser Optik ist in Richtung der Elektrode 24 gerichtet, damit der Operateur die Manipulation des Gewebes im Blick hat. Bei dieser Optik kann es sich um ein Stablinsensystem oder um eine Glasfaser handeln. Am proximalen Ende der Optik befindet sich, wie in der Fig. 1 dargestellt, ein Okular 30 oder eine Kamera.

In der Fig. 2 ist ein mögliches Ausführungsbeispiel eines Kontaktkörpers 17 dargestellt. Dieser Kontaktkörper 17 kann sowohl ringartig als auch quaderartig ausgebildet sein. Als besonders bevorzugtes Material für diesen Kontaktkörper 17 hat sich PTFE erwiesen. Allerdings sind auch andere Fluorpolymere mit ähnlichen Materialeigenschaften denkbar. PTFE bietet sich unter anderem deswegen an, da es eine besonders geringe Gleitreibung aufweist. Der Kontaktkörper 17 weist nämlich eine durchgängige Bohrung 19 auf, durch die sich im zusammengebauten Zustand des Transporteurs 11 die Optikführung 18 erstreckt. Bei der Betätigung des Transporteurs 11 wird der Kontaktkörper 17 entlang der Optikführung 18 geführt. Damit dieses von dem Operateur durchgeführte Entlangführen ohne größere Kraftanstrengung durchführbar ist, gilt es den Gleitwiderstand gering zu halten.

An einer Außenwandung weist der Kontaktkörper 17 eine Bohrung 31 auf, in die das zweite Griffmittel 16 lagerbar ist. Diese Bohrung 31 ist allerdings nicht zwingend erforderlich. Vielmehr ist denkbar, dass das zweite Griffmittel 16 auch auf eine andere Art und Weise mit dem Kontaktkörper 17 verbunden werden kann.

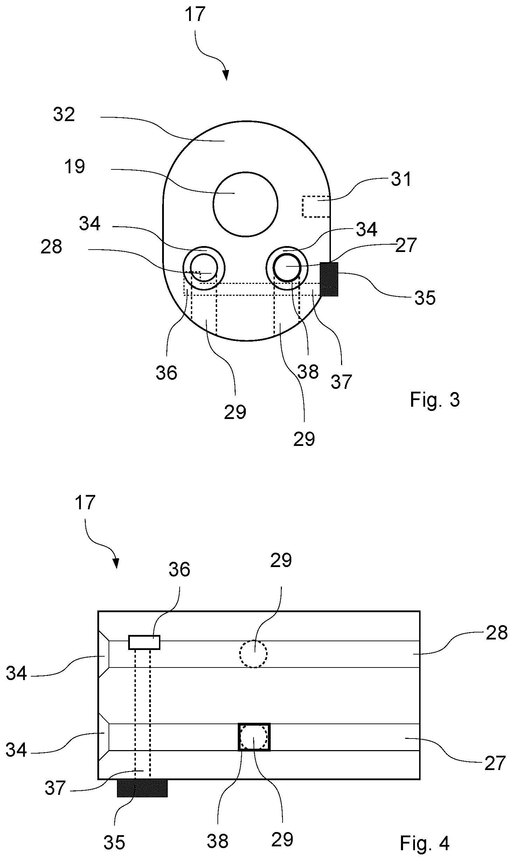

Erfindungsgemäß erstreckt sich durch den Kontaktkörper 17 eine kanalartige Aufnahme 27 und zwar von einer Stirnseite 32 zu der anderen Stirnseite 33 des Kontaktkörpers 17. Das hier dargestellte Ausführungsbeispiel des Kontaktkörpers 17 weist zwei dieser kanalartigen Aufnahmen 27, 28 auf (Fig. 3, Fig. 4). Diese beiden Aufnahmen 27, 28 sind parallel zueinander sowie parallel zu der Bohrung 19 ausgerichtet. Die Aufnahmen 27, 28 befinden sich in dem Kontaktkörper 17 in einer gleichen Ebene und sind derart voneinander beabstandet, dass die beiden proximalen Enden des Elektrodenträgers 23 in die Aufnahmen 27, 28 führbar sind. Diese beiden proximalen Enden des Elektrodenträgers 23 weisen jeweils einen elektrischen Kontakt auf, nämlich beispielsweise einen Aktiv-Kontakt und einen Return-Kontakt.

In den Aufnahmen 27, 28 werden diese Kontakte des Elektrodenträgers 23 nicht nur mechanisch fixiert sondern auch mit einem vordefinierten elektrischen Potenzial beaufschlagt. Für ein leichteres Einführen der Kontakte in die Aufnahmen 27, 28 weisen die Enden der Aufnahmen 27, 28 an der Stirnseite 32 jeweils eine trichterartige Senkung bzw. einen Trichter 34 auf. Durch diesen Trichter 34 wird das Koppeln des Elektrodenträgers 23 mit dem Kontaktkörper 17 erheblich vereinfacht.

Die Innenwandungen der Aufnahmen 27, 28 weisen elektrische Kontakte auf. Bei diesen Kontakten kann es sich beispielsweise um Lamellenkontakte oder Spiralfederkontakte handeln. Damit diese Kontakte innerhalb der Aufnahmen 27, 28 mit elektrischer Energie beaufschlagbar sind, sind beide Aufnahmen 27, 28 mit jeweils einem Steckverbinder 29, insbesondere einem zweipoligen Koaxial-Stecker bzw. einer entsprechende Buchse für einen Koaxial-Stecker, verbunden. Diese Steckverbinder 29 können, wie beispielsweise in den Fig. 2 und 3 dargestellt, von unten durch den Kontaktkörper 17 an die Aufnahmen 27, 28 herangeführt werden. Gleichermaßen ist es denkbar, dass sich der Steckverbinder 29 parallel zu den Aufnahmen 27, 28 durch den Kontaktkörper 17 erstrecken. Zur Inbetriebnahme des Resektoskopes 10 sind in den Steckverbinder 29 Stecker zu fügen, die mit einem elektrischen Leiter bzw. einem Kabel mit dem nicht dargestellten Generator verbunden sind.

Um den Elektrodenträger 23 nicht nur elektrisch in dem Kontaktkörper 17 zu kontaktieren, sondern auch um die mechanische Arretierung zu dem Kontaktkörper 17 herzustellen, ist wenigstens einer Aufnahme 28 ein Rastmittel 36 zugeordnet. Dieses Rastmittel 36 ist über einen schaftartigen Stift 37 mit einem Knopf 35 verbunden (Fig. 4). Das Rastmittel 36 ist federvorgespannt und durch den Knopf 35 betätigbar. Durch die Betätigung des Knopfes 35 wird das Rastmittel 36 aus der Aufnahme 28 heraus gedrückt, sodass der Elektrodenträger 23 mit den beiden Kontakten in den Kontaktkörper 17 einführbar ist. Der in die Aufnahme 28 geführte Kontakt weist eine Ausnehmung auf, in die das Rastmittel 36 einrastet, wodurch die mechanische Kopplung hergestellt ist. Es ist gleichermaßen denkbar, dass beim Einführen des Elektrodenträgers 23 in die Aufnahme 28 das Rastmittel 36 zurückgedrückt wird und in die entsprechende Ausnehmung einrastet.

Vorzugsweise ist das Rastmittel 36 nur einer Aufnahme 27, 28 zugeordnet, um die Gefahr eines elektrischen Kurzschlusses zwischen den beiden Aufnahmen 27, 28 zu vermeiden.

Die durchgängigen, kanalartig ausgebildeten Aufnahmen 27, 28 lassen sich besonders gut reinigen. So lässt sich eine Spülflüssigkeit auf eine einfache sowie effektive Art und Weise von einer Stirnseite 32 zu der anderen Stirnseite 33 durch den Kontaktkörper 17 hindurchspülen. Der oben beschriebene Nachteil, dass in einem Sackloch insbesondere Partikel auch nach der Reinigung verbleiben, kann hier komplett ausgeschlossen werden. Auch ein anschließendes Trocknen des Kontaktkörpers 17 ist ohne weiteres möglich.

Um einen Kriechstrom oder einen elektrischen Durchschlag zu verhindern, können Komponenten des Resektoskopes 10, welche einer Stirnseite 32, 33 des Kontaktkörpers 17 zugewandt sind, aus einem elektrisch isolierenden Material bestehen oder eine entsprechende Beschichtung aufweisen. Dadurch wird verhindert, dass beispielsweise durch Spülflüssigkeit, die während der Operation durch die Aufnahmen 27, 28 treten könnte, ein elektrischer Kontakt zu einem weiteren elektrischen Potenzial hergestellt wird.

Der Kontaktkörper 17 ist derart bemessen, dass ein Abstand zwischen einem Kontaktteil der Elektrode bzw. dem Kontakt 38 und einer der Stirnseiten 32, 33 größer ist als der Abstand zwischen der Elektrode 24 und der nächstgelegenen leitfähigen Komponente, insbesondere der Optik oder einem Elektrodenhüllrohr, auf Return-Potential. Dadurch kann sichergestellt werden, dass das Plasma an der Elektrode 24 zündet und nicht in dem Kontaktkörper 17.

**Bezugszeichenliste**

| | | | |
|---|---|---|---|
| 10 | Resektoskop | 29 | Steckverbinder |
| 11 | Transporteur | 30 | Okular |
| 12 | Schaft | 31 | Bohrung |
| 13 | Hauptkörper | 32 | Stirnseite |
| 14 | Griffeinheit | 33 | Stirnseite |
| 15 | erstes Griffmittel | 34 | Trichter |
| 16 | zweites Griffmittel | 35 | Knopf |
| 17 | Kontaktkörper | 36 | Rastmittel |
| 18 | Optikführung | 37 | Stift |
| 19 | Bohrung | 38 | elektrischer Kontakt |
| 20 | Optikführungsplatte | | |
| 21 | Federelement | | |
| 22 | Innenrohr | | |
| 23 | Elektrodenträger | | |
| 24 | Elektrode | | |
| 25 | Daumenring | | |
| 26 | Führung | | |
| 27 | Aufnahme | | |
| 28 | Aufnahme | | |

## Patentansprüche

1. Kontaktkörper (17) für ein elektrochirurgisches Handgerät, insbesondere Resektoskop (10), mit mindestens einer Aufnahme (27, 28) für ein mindestens einen elektrischen Kontakt aufweisendes proximales Ende eines Elektrodenträgers (23) des Handgerätes, wobei der Kontakt in der Aufnahme (27, 28) mechanisch sowie elektrisch koppelbar ist, **gekennzeichnet durch** zwei Aufnahmen (27, 28), die jeweils als durchgehende Bohrung durch den Kontaktkörper (17) ausgebildet sind, wobei die beiden Aufnahmen (27, 28) parallel zueinander sowie parallel zu einer Längsachse des Handgerätes ausgerichtet sind und in einer gemeinsamen Ebene liegen und mindestens eine der Aufnahmen (27, 28) an einer Innenwandung einen elektrischen Kontakt (38) aufweist, der über einen Steckverbinder (29) und eine elektrische Verbindung mit einem Generator verbindbar ist, wobei der Steckverbinder (29) vorzugsweise in den Kontaktkörper (17) integriert ist.

2. Kontaktkörper (17) nach Anspruch 1, **dadurch gekennzeichnet, dass** der elektrische Kontakt (38) innerhalb der mindestens einen Aufnahme (27, 28) als zylinderförmiger, hohlzylinder- oder ringförmiger elektrischer Kontakt, vorzugsweise als Lammellenkontakt oder Spiralfederkontakt, ausgebildet ist.

3. Kontaktkörper (17) nach Anspruch 1 oder 2, **dadurch gekennzeichnet, dass** ein Abstand des mindestens einen Kontaktes (38) zu einer Stirnseite (32, 33) des Kontaktkörpers (17), insbesondere zu einem Austritt der mindestens einen Aufnahme (27, 28), mindestens 2 mm, bevorzugst mindestens 4 mm, insbesondere mindestens 6 mm, beträgt.

4. Kontaktkörper (17) nach einem der vorhergehenden Ansprüche, **dadurch gekennzeichnet, dass** sich in mindestens eine Aufnahme (27, 28) ein mechanisches Rastmittel (36) erstreckt zur lösbaren Kopplung mindestens eines proximalen Endes des Elektrodenträgers (23), wobei das Rastelement (36) über ein Handhabungsmittel, insbesondere einen federvorgespannten Knopf (35), betätigbar, vorzugsweise arretierbar und/oder lösbar, ist.

5. Kontaktkörper (17) nach einem der vorhergehenden Ansprüche, **dadurch gekennzeichnet, dass** die Bohrung der mindestens einen Aufnahme (27, 28) an einem distalen Ausgang trichterartig ausgebildet ist, insbesondere dass eine Stirnseite (32) des Kontaktkörpers (17) um die Bohrung der mindestens einen Aufnahme (27, 28) eine trichterartige Senkung bzw. einen Trichter (34) aufweist.

6. Kontaktkörper (17) nach einem der vorhergehenden Ansprüche, **dadurch gekennzeichnet, dass** in der Bohrung vor oder hinter dem elektrischen Kontakt (38) eine Dichtung, insbesondere eine Fluiddichtung, angeordnet ist.

7. Kontaktkörper (17) für ein elektrochirurgisches Handgerät nach einem der vorangegangenen Ansprüche, **dadurch gekennzeichnet, dass** der Kontaktkörper (17) aus Kunststoff, insbesondere PTFE, PFA, einem anderen Fluorpolymer oder PEEK hergestellt ist.

8. Elektrochirurgisches Handgerät, insbesondere Resektoskop (10), mit einem Elektrodenträger (23), der an einem distalen Ende eine Elektrode (24) aufweist und an einem proximalen Ende mindestens einen elektrischen Kontakt, mit einer Griffeinheit (14), bestehend aus einem ersten Griffmittel (15) und einem zweiten Griffmittel (16), mit einem rohrartigen Schaft (12), der mit einem proximalen Ende an dem ersten Griffmittel (15) gekoppelt ist, mit einer Optikführung (18) zur Aufnahme einer Optik und einem Kontaktkörper (17), durch den die Optikführung (18) führbar ist, das zweite Griffmittel (16) befestigbar ist und in dem der mindestens eine elektrische Kontakt des Elektrodenträgers (23) verrastbar und/oder elektrisch kontaktierbar ist, **gekennzeichnet durch** einen Kontaktkörper (17) nach mindestens einem der Ansprüche 1 bis 7.

9. Elektrochirurgisches Handgerät nach Anspruch 8, **dadurch gekennzeichnet, dass** wenigstens eine Komponente, insbesondere ein Anschlag, des Handgerätes, der in proximaler oder distaler Richtung an dem Kontaktkörper (17) angeordnet ist oder an dem Kontaktkörper (17) anschlägt, aus einem elektrisch nicht leitenden Material besteht oder eine elektrische nicht leitende Schicht, vorzugsweise Beschichtung, aufweist.

10. Elektrochirurgisches Handgerät nach Anspruch 8 oder 9, **dadurch gekennzeichnet, dass** eine elektrisch leitfähige Komponente, insbesondere ein Anschlag, des Handgerätes durch einen Luftspalt von dem Kontaktkörper (17), vorzugsweise einem Austritt mindestens einer kanalartigen Aufnahme (27, 28) des Kontaktkörpers (17), getrennt ist, wobei der Luftspalt eine Breite von mindestens 1 mm, insbesondere mindestens 2 mm, aufweist.

11. Elektrochirurgisches Handgerät nach einem der Ansprüche 8 bis 10, **dadurch gekennzeichnet, dass** ein Abstand zwischen der Elektrode (24) und einem distalen Ende des Schaftes (12), insbesondere der Optik oder einem Elektrodenhüllrohr, geringer ist als ein Abstand zwischen einem proximalen Ende des Elektrodenträgers (23) und einer proximalen Stirnseite (33) des Kontaktkörpers (17) und/oder geringer ist als ein Abstand zwischen einem elektrischen Kontakt (38) und wenigstens einer Stirnseite (32, 33) des Kontaktkörpers (17).

## Claims

1. A contact body (17) for an electrosurgical handheld device, in particular a resectoscope (10), with at least one receptacle (27, 28) for a proximal end, having at least one electrical contact, of an electrode carrier (23) of the handheld device, wherein the contact is able to be mechanically and electrically coupled in the receptacle (27, 28), **characterized by** two receptacles (27, 28) each designed as a continuous bore through the contact body (17), wherein the two receptacles (27, 28) are oriented parallel to each other and also parallel to a longitudinal axis of the handheld device and lie in a common plane and at least one of the receptacles (27, 28) has, on an inner wall, an electrical contact (38) which is connectable to a generator via a plug connector (29) and an electrical connection, wherein the plug connector (29) is preferably integrated in the contact body (17).

2. The contact body (17) as claimed in claim 1, **characterized in that** the electrical contact (38) is designed, inside the at least one receptacle (27, 28), as a cylindrical, hollow cylindrical or annular electrical contact, preferably as a lamella contact or helical spring contact.

3. The contact body (17) as claimed in claim 1 or 2, **characterized in that** a distance of the at least one contact (38) from an end face (32, 33) of the contact body (17), in particular from an outlet of the at least one receptacle (27, 28), measures at least 2 mm, preferably at least 4 mm, in particular at least 6 mm.

4. The contact body (17) as claimed in one of the preceding claims, **characterized in that** a mechanical latching means (36), for releasably coupling at least a proximal end of the electrode carrier (23), extends into at least one receptacle (27, 28), wherein the latching element (36) is able to be actuated, preferably locked and/or released, via a manoeuvering means, in particular a spring-pretensioned button (35).

5. The contact body (17) as claimed in one of the preceding claims, **characterized in that** the bore of the at least one receptacle (27, 28) has a funnel-like shape at a distal outlet, in particular **in that** an end face (32) of the contact body (17) has a funnel-like depression or a funnel (34) around the bore of the at least one receptacle (27, 28).

6. The contact body (17) as claimed in one of the preceding claims, **characterized in that** a seal, in particular a fluid seal, is arranged in the bore, in front of or behind the electrical contact (38).

7. The contact body (17) for an electrosurgical handheld device as claimed in one of the preceding claims, **characterized in that** the contact body (17) is made of plastic, in particular PTFE, PFA, another fluoropolymer or PEEK.

8. An electrosurgical handheld device, in particular a resectoscope (10), with an electrode carrier (23) which at a distal end has an electrode (24) and at a proximal end has at least one electrical contact, with a grip unit (14) comprising a first gripping means (15) and a second gripping means (16), with a tubular shaft (12) which is coupled with a proximal end to the first gripping means (15), with an optical guide (18) for receiving an optical unit, and a contact body (17) through which the optical guide (18) can be guided, the second gripping means (16) can be fastened, and in which the at least one electrical contact of the electrode carrier (23) can be latched and/or electrically contacted, **characterized by** a contact body (17) as claimed in at least one of claims 1 through 7.

9. The electrosurgical handheld device as claimed in claim 8, **characterized in that** at least one component, in particular a stop, of the handheld device, which is arranged in the proximal or distal direction to the contact body (17) or strikes the contact body (17), is made of an electrically non-conductive material or has an electrically non-conductive layer, preferably a coating.

10. The electrosurgical handheld device as claimed in claim 8 or 9, **characterized in that** an electrically conductive component, in particular a stop, of the handheld device is separated by an air gap from the contact body (17), preferably from an outlet of at least one channel-like receptacle (27, 28) of the contact body (17), wherein the air gap has a width of at least 1 mm, in particular at least 2 mm.

11. The electrosurgical handheld device as claimed in one of claims 8 through 10, **characterized in that** a distance between the electrode (24) and a distal end of the shaft (12), in particular the optical unit or an electrode casing tube, is less than a distance between a proximal end of the electrode carrier (23) and a proximal end face (33) of the contact body (17) and/or less than a distance between an electrical contact (38) and at least one end face (32, 33) of the contact body (17).

## Revendications

1. Corps de contact (17) pour un appareil à main électrochirurgical, en particulier un résectoscope (10), présentant au moins un logement (27, 28) pour une extrémité proximale, présentant au moins un contact électrique, d'un support d'électrode (23) de l'appareil à main, le contact dans le logement (27, 28) pouvant être couplé tant mécaniquement qu'électriquement, **caractérisé par** deux logements (27, 28) qui sont conçus chacun en tant que trou passant à travers le corps de contact (17), les deux logements (27, 28) étant orientés parallèlement l'un à l'autre ainsi que parallèlement à un axe longitudinal de l'appareil à main et situés dans un plan commun et au moins l'un des logements (27, 28) présentant un contact électrique (38) au niveau d'une paroi interne, lequel contact peut être connecté par l'intermédiaire d'un connecteur (29) et d'une connexion électrique à un générateur, le connecteur (29) étant de préférence intégré dans le corps de contact (17).

2. Corps de contact (17) selon la revendication 1, **caractérisé en ce que** le contact électrique (38) dans ledit au moins un logement (27, 28) est conçu comme un contact électrique en forme de cylindre, en forme de cylindre creux ou en forme d'anneau, de préférence sous forme de contact à lamelles ou de contact à ressort en spirale.

3. Corps de contact (17) selon la revendication 1 ou 2, **caractérisé en ce qu'**une distance dudit au moins un contact (38) par rapport à une face frontale (32, 33) du corps de contact (17), en particulier à une sortie dudit au moins un logement (27, 28), représente au moins 2 mm, de préférence au moins 4 mm, en particulier au moins 6 mm.

4. Corps de contact (17) selon l'une des revendications précédentes, **caractérisé en ce qu'**un moyen d'encliquetage mécanique (36) s'étend dans ledit au moins un logement (27, 28) pour un accouplement amovible d'au moins une extrémité proximale du support d'électrode (23), l'élément d'encliquetage (36) pouvant être actionné, de préférence bloqué et/ou désaccouplé, par l'intermédiaire d'un moyen de manipulation, en particulier d'un bouton (35) précontraint par un ressort.

5. Corps de contact (17) selon l'une des revendications précédentes, **caractérisé en ce que** le trou dudit au moins un logement (27, 28) est conçu sous forme d'entonnoir au niveau d'une sortie distale, en particulier **en ce qu'**une face frontale (32) du corps de contact (17) présente autour du trou dudit au moins un logement (27, 28) un abaissement en forme d'entonnoir ou, selon le cas, un entonnoir (34).

6. Corps de contact (17) selon l'une des revendications précédentes, **caractérisé en ce qu'**un joint, en particulier un joint étanche aux fluides, est agencé dans le trou devant ou derrière le contact électrique (38).

7. Corps de contact (17) pour un appareil à main électrochirurgical selon l'une des revendications précédentes, **caractérisé en ce que** le corps de contact (17) est fabriqué en un matériau synthétique, en particulier en PTFE, PFA, un autre polymère fluoré ou en PEEK.

8. Appareil à main électrochirurgical, en particulier un résectoscope (10), comportant un support d'électrode (23) qui présente une électrode (24) à une extrémité distale et au moins un contact électrique à une extrémité proximale, comportant une unité de préhension (14) constituée d'un premier moyen de préhension (15) et d'un second moyen de préhension (16), comportant une tige tubulaire (12), qui est accouplée par une extrémité proximale au premier moyen de préhension (15), comportant un guide optique (18) destiné à recevoir une optique et un corps de contact (17) à travers lequel le guide optique (18) peut être guidé, auquel le deuxième moyen de préhension (16) peut être fixé et dans lequel ledit au moins un contact électrique du support d'électrode (23) peut être encliqueté et/ou mis en contact électrique, **caractérisé par** un corps de contact (17) selon au moins l'une quelconque des revendications 1 à 7.

9. Appareil à main électrochirurgical selon la revendication 8, **caractérisé en ce qu'**au moins un composant, en particulier une butée, de l'appareil à main, qui est agencé sur le corps de contact (17) dans le sens proximal ou distal ou qui bute contre le corps de contact (17), est constitué par un matériau électriquement non conducteur ou présente une couche électriquement non conductrice, de préférence un revêtement.

10. Appareil à main électrochirurgical selon la revendication 8 ou 9, **caractérisé en ce qu'**un composant électriquement conducteur, en particulier une butée, de l'appareil à main est séparé par un entrefer du corps de contact (17), de préférence d'une sortie d'au moins un logement (27, 28) de type canal du corps de contact (17), l'entrefer présentant une largeur d'au moins 1 mm, en particulier d'au moins 2 mm.

11. Appareil à main électrochirurgical selon l'une des revendications 8 à 10, **caractérisé en ce qu'**une distance entre l'électrode (24) et une extrémité distale de la tige (12), en particulier de l'optique ou d'une gaine d'électrode, est inférieure à une distance entre une extrémité proximale du support d'électrode (23) et une face frontale proximale (33) du corps de contact (17) et/ou inférieure à une distance entre un contact électrique (38) et au moins une face frontale (32, 33) du corps de contact (17).
